# EUROPEAN PATENT APPLICATION

(11) **EP 1 598 427 A1**
(43) Date of publication of application: **23.11.2005**
(21) Application number: 04011664.2
(22) Date of filing: 17.05.2004
(51) Int. Cl.: C12Q 1/68

(54) **Method for the amplification of a target nucleic acid**

(71) Applicant: Charité - Universitätsmedizin Berlin, 10117 Berlin (DE)
(72) Inventor: Volz, Armin, 14050 Berlin (DE); Wende, Hagen, 10829 Berlin (DE)
(74) Representative: Bohmann, Armin K.

(57) **Abstract**

The present invention is related to a method for the amplification of a target nucleic acid, whereby the method comprises the following steps:
a) providing the target nucleic acid;
b) providing a first primer, whereby the primer is at least partially complementary to a part of the target nucleic acid;
c) providing a polymerase;
d) reacting the target nucleic acid, the primer and the polymerase providing a reaction product, whereby the reaction product comprises a restriction site for a restriction endonuclease;
e) digesting the reaction product of step d) with the restriction endonuclease providing a digestion product;
f) ligating the 5' end of the first primer with the digestion product producing a circular target;
g) providing a second primer, whereby the second primer is at least partially identical to the target nucleic acid;
h) reacting the circular target with the second primer and a second polymerase providing a first amplification product;
i) optionally providing a third primer, whereby the sequence of the third primer is at least partially identical to a part of the first primer or complementary to the target nucleic acid; and
j) optionally reacting the second and the third primer with the first amplification product or the circular target and a third polymerase to provide a series of second amplification products.

## Description

The present invention is related to methods for the amplification of a target nucleic acid and methods comprising an amplification step such as methods for the detection of polymorphisms, methods for diagnosis, methods for phase determination, methods for genotyping, methods for genomic mapping, methods for DNA sequencing and methods for synthesis of DNA probes.

Nucleic acid amplification technology has increased the ability to ask detailed questions about genotype or transcriptional phenotype in small biological samples and has provided the impetus for many significant advances in biology, especially in the field of genetics. One method for amplifying nucleic acid and deoxyribonucleic acid in particular, is the so called rolling circle amplification (RCA). This method is based on the finding that DNA circles can be copied by a rolling circle replication mechanism which basically is known from the replication of phage genomes. RCA is an isothermal process for generating multiple copies of the sequences to be amplified. In rolling circle DNA replication *in vivo* a DNA polymerase extends a primer on a circular template (Komberg, A. and Baker T. A., DNA Replication, W. H. Freeman, New York 1991). The amplification products consist of tandemly linked copies of the complementary sequence of the template. RCA is a method that has been adapted for use *in vitro* for DNA amplification. Zhang et al. (Zhang D. Y. Gene, 274 (2001) 209-2016) describe a DNA amplification technique which is termed ramification amplification (RAM), whereby a circular probe or primer is used that is covalently linked by a DNA ligase when it hybridises to a target. A DNA polymerase extends a bound forward primer along the circular probe and continuously displaces a downstream strand, generating a multimeric single-stranded DNA (ssDNA) which is analogous to the afore-described *in vivo* "rolling circle" replication of bacteriophages. This multimeric ssDNA then serves as a template for multiple reverse primers to hybridize. The reverse primers are also extended by the DNA polymerase, and downstream DNA is again displaced, generating another multimeric product where the initially used forward primers hybridize again and initiate another round of amplification. In total, a large ramified (branching) DNA complex is produced, resulting in an exponential amplification.

Nevertheless, any method of the prior art is facing a limitation in either specificity of the amplification and thus, if used in connection with an analytical method, in sensitivity in detection of a specific target nucleic acid. A further disadvantage of the methods according to the prior art is the substantial generation of undesired by-products which add to the aforementioned problem of unspecific amplification and loss in sensitivity.

The problem underlying the present invention was thus to provide a method for the amplification of a target nucleic acid which is highly specific in terms of selectivity of the nucleic acid to be amplified as well as in terms of specificity of the amplified nucleic acid, i.e. that the amplified nucleic acid still corresponds to the nucleic acid to be amplified in terms of sequence identity.

In a further aspect the problem underlying the present invention is to provide methods comprising an amplification step such as methods for the detection of polymorphisms, methods for diagnosis, methods for phase determination, methods for genotyping, methods for genomic mapping, methods for DNA sequencing and methods for the synthesis of DNA and DNA probes in particular, whereby such methods are more accurate in terms of efficiency, selectivity and specificity as defined herein.

These and other objections are solved in a first aspect by a method for the amplification of a target nucleic acid, whereby the method comprises the following steps:
a) providing the target nucleic acid;
b) providing a first primer, whereby the primer is at least partially complementary to a part of the target nucleic acid;
c) providing a polymerase;
d) reacting the target nucleic acid, the primer and the polymerase providing a reaction product, whereby the reaction product comprises a restriction site for a restriction endonuclease;
e) digesting the reaction product of step d) with the restriction endonuclease providing a digestion product;
f) ligating the 5' end of the first primer with the digestion product producing a circular target;
g) providing a second primer, whereby the second primer is at least partially identical to the target nucleic acid;
h) reacting the circular target with the second primer and a second polymerase providing a first amplification product;
i) optionally providing a third primer, whereby the sequence of the third primer is at least partially identical to a part of the first primer or complementary to the target nucleic acid; and
j) optionally reacting the second and the third primer with the first amplification product or the circular target and a third polymerase to provide a series of second amplification products.

In an embodiment the 5'-end of the first primer is at least partially complementary to the 5' overhang of the digestion product of step e)

In an embodiment the 5' end of the first primer is ligated to the 3' end of the reaction product generated in steps d) and e).

In an embodiment the restriction enzyme generates a 5' overhang.

The problem underlying the present invention is solved in a second aspect by a method for the amplification of a target nucleic acid, whereby the method comprises the following steps:
a) providing the target nucleic acid;
b) providing a first primer, whereby the primer is at least partially complementary to a part of the target nucleic acid;
c) providing a polymerase;
d) reacting the target nucleic acid, the primer and the polymerase providing a reaction product, whereby the reaction product comprises a restriction site for a restriction endonuclease;
e) digesting the reaction product of step d) with the restriction endonuclease providing a digestion product;
f) providing an extension oligonucleotide, whereby one end of the extension oligonucleotide is at least partially complementary to the 3' overhang of the digestion product of step e) annealing such extension oligonucleotide to the digestion product of step e), providing an annealed digestion product;
g) ligating the 5' end and 3' end of the annealed digestion product producing a circular target;
h) providing a second primer, whereby the second primer is at least partially identical to the target nucleic acid;
i) reacting the circular target with the second primer and a second polymerase providing a first amplification product;
j) optionally providing a third primer, whereby the sequence of the third primer is at least partially identical to a part of the first primer or complementary to the target nucleic acid; and
k) optionally reacting the second and the third primer with the first amplification product or the circular target and a third polymerase to provide a series of second amplification products.

In an embodiment the restriction enzyme generates a 3' overhang.

In an embodiment the 5' end of the first primer is at least partially complementary to a stretch, preferably an end, more preferably the 3' end of the extension oligonucleotide.

In an embodiment of the first and the second aspect the first primer comprises 30 to 400 nucleotides, preferably 50 to 80 nucleotides.

In an embodiment of the first and the second aspect the circular target comprises about 80 to 150 nucleotides.

In an embodiment of the first and the second aspect the 3' end of the first primer is at least complementary to 15 -25 bp of the target nucleic acid.

In an embodiment of the first and the second aspect the extension oligonucleotide comprises about 12 to 30 nucleotides, preferably 15 to 20 nucleotides.

In an embodiment of the first and the second aspect the second primer and/or the third primer comprises 15 to 25 nucleotides of the target nucleic acid, preferably 20 nucleotides or a complementary sequence thereto.

The problem underlying the present invention is solved in a third aspect by a method for the amplification of a target nucleic acid comprising the following steps:
a) providing the target nucleic acid;
b) providing an extension oligonucleotide;
c) providing a first mediating primer, whereby the first mediating primer is at least partially complementary to a first stretch of the target nucleic acid and at least partially complementary to a first stretch of the extension oligonucleotide;
d) providing a second mediating primer, whereby the second mediating primer is at least partially complementary to a second stretch of the target nucleic acid and at least partially complementary to a second stretch of the extension oligonucleotide;
e) reacting the extension oligonucleotide, the first mediating primer and the second mediating primer and the target nucleic acid with a ligase creating a circular ligation product of the target nucleic acid and the first primer;
f) providing a first primer, whereby the first primer is at least partially complementary to the target nucleic acid;
g) reacting the circular ligation product with the first primer and a first polymerase providing a first amplification product;
h) optionally providing a second primer, whereby the sequence of the second primer is at least partially identical to a part of the extension oligonucleotide or of the target nucleic acid; and
i) reacting the first and second primer with the first amplification product or the circular ligation product and a second polymerase to provide a series of second amplification products.

In an embodiment of the third aspect the extension oligonucleotide comprises 30 to 400 nucleotides, preferably 50 to 80 nucleotides.

In an embodiment of the third aspect the target nucleic acid is created by cutting of a nucleic acid molecule with one or several restriction enzymes and subsequent denaturation, whereby the target nucleic acid preferably comprises about 40 to 150 nucleotides.

The problem underlying the present invention is solved in a fourth aspect by a method for the amplification of a target nucleic acid comprising the following steps:
a) providing a target nucleic acid;
b) providing a mediating primer, whereby the first end of the mediating primer is at least partially complementary to the first end of the target nucleic acid and the second end of the mediating primer is at least partially complementary to the second end of the target nucleic acid;
c) reacting the target nucleic acid and the mediating primer with a ligase providing a circular ligation product;
d) reacting the circular ligation product with a DNA polymerase, whereby the mediating primer is annealed to the circular ligation product providing an amplification product;
e) optionally providing a second primer, whereby the sequence of the second primer is at least partially identical to the target nucleic acid; and
f) reacting the mediating and second primer with the amplification product or the ligation product and a first polymerase to provide a series of second amplification products.

In an embodiment of the fourth aspect the target nucleic acid comprises about 40 to 500 nucleotides, preferably 80 to 150 nucleotides, and more preferably about 100 nucleotides.

In an embodiment of the fourth aspect the mediating primer comprises about 15 to 35 bp nucleotides, preferably 20 nucleotides.

In an embodiment of the first, second, third and fourth aspect the target nucleic acid is a deoxyribonucleic acid.

In an embodiment of the first, second, third and fourth aspect the target nucleic acid as used in step a) is a single stranded nucleic acid.

In an embodiment of the first, second, third and fourth aspect any of the primers, mediating primers and/or extension oligonucleotides used comprises a label.

In a preferred embodiment of the first, second, third and fourth aspect the label is selected from the group comprising radiolabels and fluorescence labels

In a more preferred embodiment of the first, second, third and fourth aspect the label is a combination of a quenching group and a fluorophore, whereby preferably the quenching functionality being inherent to the primer(s).

In a most preferred embodiment of the first, second, third and fourth aspect the quenching group and the fluorophore are attached or inherent to the primer in a spatially separated manner, whereby upon ligation of the primer with or annealing of the primer to the target nucleic acid, ligation product and/or amplification product, said primer exhibits a fluorescence different from the fluorescence shown by the primer in an un-ligated and/or un-annealed form.

In an embodiment of the first, second, third and fourth aspect the method is performed *in vitro.*

In an embodiment of the first, second, third and fourth aspect the sequence of steps in its entirety or parts thereof are carried out as an isothermal process.

In an embodiment of the first, second, third and fourth aspect any of the first, second and third polymerase is a DNA polymerase.

In a preferred embodiment of the first, second, third and fourth aspect the DNA polymerase is selected from the group comprising strand displacing DNA polymerase III, pol III-like DNA polymerases, and pol I-type DNA polymerases.

In an embodiment of the first, second, third and fourth aspect the target nucleic acid is derived from a ribonucleic acid by the following steps:
a) providing a ribonucleic acid, preferably an mRNA;
b) providing a primer which is at least partially complementary to the ribonucleic acid of step a);
c) reacting the ribonucleic acid and the primer with a reverse transcriptase providing a reverse transcription product; and
d) removing the RNA moiety of the reverse transcription product, preferably by RNAse H digestion, providing a single stranded deoxyribonucleic acid.

In a preferred embodiment of the first, second, third and fourth aspect the single stranded deoxyribonucleic acid is used as the target nucleic acid.

In an alternative embodiment of the first, second, third and fourth aspect the method further comprises the following steps:
e) providing a further primer which anneals at least partially to the single stranded deoxyribonucleic acid;
f) performing a DNA synthesis creating a double stranded nucleic acid; and
g) using one strand of the double stranded nucleic acid as the target nucleic acid.

The problem underlying the present invention is solved in a fifth aspect by a method for the detection of polymorphisms comprising an amplification process, whereby the amplification process comprises the method according to any of the first, second, third and/or fourth aspect.

The problem underlying the present invention is solved in a sixth aspect by a method for diagnosis comprising an amplification process, whereby the amplification process comprises the method according to any of the first, second, third and/or fourth aspect.

The problem underlying the present invention is solved in a seventh aspect by a method for phase determination comprising an amplification process, whereby the amplification process comprises the method according to any of the first, second, third and/or fourth aspect.

The problem underlying the present invention is solved in an eighth aspect by a method for genotyping comprising an amplification process, whereby the amplification process comprises the method according to any of the first, second, third and/or fourth aspect.

The problem underlying the present invention is solved in a ninth aspect by a method for genomic mapping comprising an amplification process, whereby the amplification process comprises the method according to any of the first, second, third and/or fourth aspect.

The problem underlying the present invention is solved in a tenth aspect by a method for DNA sequencing comprising an amplification process, whereby the amplification process comprises the method according to any of the first, second, third and/or fourth aspect.

The problem underlying the present invention is solved in an eleventh aspect by a method for synthesis of DNA probes comprising an amplification process, whereby the amplification process comprises the method according to any of the first, second, third and/or fourth aspect.

The problem underlying the present invention is solved in a twelfth aspect by a method for cloning comprising an amplification process, whereby the amplification process comprises the method according to any of the first, second, third and/or fourth aspect.

Without wishing to be bound by any theory, the present inventor has surprisingly found that rolling circle amplification of nucleic acid can be made more specific and allows for the use of smaller amounts of target nucleic acid by including the original target or a copy thereof into an artificially generated circle which is also referred to herein as the circularized target or circular ligation product. It is within the present invention that such circle consists either of the sequence of the target nucleic acid or consists of the sequence which is essentially complementary, preferably complementary to the sequence of the target nucleic acid. As preferably used herein, the term circular ligation product refers to a circular oligonucleotide or a circular nucleic acid which comprises the target nucleic acid in full or in part, optionally together with a further stretch of nucleotides. Such further stretch of nucleotides can be derived from any oligonucleotide or part thereof or complementary sequence thereof which is selected from the group comprising any first primer, any second primer, any third primer, any extension oligonucleotide, any mediating primer, any first mediating primer, and any second mediating primer. As preferably used herein, the term circular target refers to a circular oligonucleotide or a circular nucleic acid which comprises a sequence which is complementary to a part of or the full sequence of the target nucleic acid, optionally together with a further stretch of nucleotides. Such further stretch of oligonucleotides can be derived from any oligonucleotide or part thereof or complementary sequence thereof which is selected from the group comprising any first primer, any second primer, any third primer, any extension oligonucleotide, any mediating primer, any first mediating primer, and any second mediating primer.

As used herein and if not indicated to the contrary, the term primer preferably comprises any first primer, second primer, third primer, mediating primer, first mediating primer and second mediating primer. As also used herein and if not indicated to the contrary, the term mediating primer preferably comprises any mediating primer, any first mediating primer and any second mediating primer. This circular target or circular ligation product contains necessarily some part(s) of the original target nucleic acid or a copy thereof or a nucleic acid the sequence of which is complementary thereto, which provides for the specificity or uniqueness of the nucleic acid to be amplified. As used herein the terms target nucleic acid, some part thereof, i.e. some part of the target nucleic acid, and copy of the target nucleic acid shall be used in preferred embodiments in an interchangeable manner, if not indicated to the contrary.

In addition to the target nucleic acid, the circular target or circular ligation product may contain, as outlined above, other sequences such as a further stretch of nucleotides, preferably from synthetic oligonulceotides or from further target nucleic acids. The further stretch of oligonucleotides is preferably used as a primer such as the first primer in case of the method according to the first and second aspect of the present invention for the initiation of the copying process, but may serve in other embodiments for different purposes i.e. for the simultaneous detection of a second different target molecule, as structural element adjusting the size of the circularized target or as annealing site for the second or third amplification primer.

The specificity or uniqueness of the target nucleic acid is also reflected by the second and /or third primer, the first mediating primer, the second mediating primer and the mediating primer as used herein which may act in preferred embodiments and are also referred to herein as forward/reverse primer, respectively, if not indicated to the contrary. These primers hybridize to, or anneal with the circular target or the circular ligation product at that part thereof which reflects the target nucleic acid and which is in a preferred embodiment characteristic for the target nucleic acid, i.e. reflects or confers the uniqueness to the target nucleic acid and provides for the selective amplification of the target nucleic acid. In preferred embodiments of each aspect of the present invention the second primer has a sequence which is identical or complementary to the target nucleic acid. In a further embodiment, the sequence of the second primer may comprise further elements which reflect one or several nucleotides of the circular target and the circular ligation product, respectively. The extent to which the second primer is identical to the target nucleic acid depends on the length requirements to confer a selective binding, i.e. hybridization of the second primer. The design of the second primer also depends on the particular circumstances of the reaction such as salt conditions, in particular Mg⁺⁺ concentration, melting point changing additives, primer and dNTP concentration, buffer and temperature optimum of the employed polymerase as known to the one skilled in the art. Due to this design of both the method and any type of the primers and extension oligonucleotides used herein, any by-products which might be generated due to the unspecificity of the ligase reaction during circularization of the linear components of the circular target and circular ligation product, respectively, by ligating the respective 5'- and the 3'- terminal nucleotides to each other, are not subject to later amplification, since these by-products do not contain the unique target sequence of the target nucleic acid. Additionally, due to this design of the method, smaller circles not including the target sequence, which are generated by the ligation of the first primer, as used in the first and second aspect of the method according to the present invention, which would overtake the amplification of the correct circular targets which are larger in size, are not subject to amplification, because the uniqueness conferring sequence of the target nucleic acid is not present. Said smaller circular, undesired products amount to a wrong positive signal in any detection step. As in the methods according to the present invention, only the desired circular target or circular ligation product comprising the target nucleic acid and in particular at least that part of the target nucleic acid which accounts for the specificity or uniqueness of the target nucleic acid, is amplified. Other measures to avoid false positive results in any detection steps following the amplification process such as separating the products by gel electrophoresis or verification of the products by Southern blot analysis, restriction digest or PCR amplification are thus not necessary. The PCR used in the respective methods of the prior art is typically carried out to preamplify the target nucleic acid in order to avoid the afore-described overgrowth or dominance of smaller rings of the circularized first primer which may exhibit only part of the target nucleic acid, if any.

A further advantage of the methods according to the present invention resides in the fact that even the incorporation of a single mismatch or an inappropriate primer or extension oligonucleotide into a nucleic acid to be amplified is not detrimental to the result of the analysis. This characteristic is inherent to the rolling circle amplification process, since a primer binding site generated by a wrongly inserted primer or extension oligonucleotide represents only a single invalid binding site, while amplification is carried on by the elongation of primers, annealing to a variety of correct binding sites present in each single first amplification product. Thus, the exponential increase of wrong amplification products as observed in the polymerase chain reaction is avoided. This effect is further strengthened by the fact that during the reaction any primer can bind only once to the utmost 5'-end of any rolling circle amplification product, since the resulting reaction product is double-stranded so that no further primers can bind. Therefore, the undesired reaction products are permanently removed from the reaction system. This allows discriminating the incorrect amplification products from the desired, i.e. correct amplification products. Particularly in connection with single nucleotide polymorphisms, this characteristic of the methods according to the present invention turns out to be extremely advantageous. Due to this approach, it is possible to detect as little as a single copy of the desired target nucleic acid, even if a thousand to millionfold excess of highly homologous DNA, i.e. wild-type molecules is present in a sample.

The amount of the target nucleic acid, of any primer and of any extension oligonucleotide can vary in a wide range depending on the particularities of the individual amplification process. The particular design is within the skills of the ones of the art in view of the present disclosure. It is therefore within the present invention that the circular target and circular ligation product, respectively, comprises a target nucleic acid or a sequence which is complementary thereto, which comprises more nucleotides than would be necessary to specifically address or detect the target nucleic acid, particularly that part of the target nucleic acid which confers uniqueness to the target nucleic acid. This unique part of the circular target shall not be included or complementary to any added DNA but the target itself and any specificity conferring primers or extension oligonucleotides. Such detection can be in situ, in vivo, in vitro or in any artificial settings. Therefore, the specificity requirement depends, among others, on other nucleic acids present in the system subject to the analysis setting or the reaction system where the amplification process is run. If, as realized in preferred embodiments, the analysis of a distinct gene is carried out starting from, e. g., a biopsy sample, such sample will typically comprise genomic DNA and thus the length of the part of the target nucleic acid which provides for the specificity of the particular gene is typically in the range of 20 to 40 nucleotides. Therefore, the circular target and circular ligation product, respectively, should comprise at least a stretch of 20 nucleotides reflecting that part of the target nucleic acid which provides for the uniqueness or for the selective detection of said target nucleic acid. However, it is within the present invention, that additional or less nucleotides are necessary or sufficient in preferred embodiments, preferably at either end or both ends of the respective basic target nucleic acid.

As used herein, the term that a nucleic acid, any primer or any extension oligonucleotide or any nucleotide thereof refers to a nucleic acid sequence or refers to a target nucleic acid or a nucleotide thereof shall mean in a preferred embodiment that the sequence of said nucleic acid, of any of said primer or any of said extension oligonucleotide is identical to said nucleic acid sequence or is identical to said target nucleic acid or a nucleotide or each thereof. In an alternative preferred embodiment this term shall mean that the sequence of said nucleic acid, of any of said primer or any of said extension oligonucleotide is complementary to said nucleic acid sequence or is complementary to said target nucleic acid or a nucleotide of each thereof.

In the method according to the first and second aspect of the present invention for amplifying a target nucleic acid a first primer is provided. This first primer is preferably a linear nucleic acid, more preferably a single-stranded oligonucleotide. The length of the oligonucleotide is basically not limited. However, the minimum size is such as to allow the formation of a rolling circle upon insertion of a part of the target nucleic acid as described herein. The first primer comprises preferably at its 3'-end a sequence which is complementary to the target nucleic acid, more preferably to a part of said target nucleic acid. Said part of the nucleic acid is again preferably any part of the nucleic acid conferring specificity or uniqueness to the target nucleic acid as discussed above. Upon annealing to the target nucleic acid, more particularly to a single-stranded version thereof, the first primer is extended at its 3'-end through a polymerase. The thus created reaction product comprises a restriction site for a restriction endonuclease. The thus formed double-stranded reaction product of target nucleic acid and first primer is subsequently cleaved by a restriction enzyme. Both the synthesis of the double strand starting from the 3'-end of the first primer, as well as the digestion of the thus formed double-stranded structure by restriction endonucleases are known to the one skilled in the art. Among others, part of the performing of the respective step is to provide the respective enzymatic activities, buffers, nucleotides and the like.

The 5' end of the first primer which has not been elongated by the polymerase reaction is designed in a way that it hybridizes preferably to the single stranded 5' overhang of the double-stranded structure which has been created by the restriction enzyme. The sequence complementarity shown by the non-extended end of the first primer can be formed by a recognition site or cleavage site for a restriction enzyme, preferably to the extent that such recognition site or cleavage site is reflected on a single-stranded nucleic acid. If a restriction enzyme with a 3' single stranded overhang is used for the digestion of the first reaction product, the hybridization may be mediated by an additional mediating primer, also referred to herein as extension oligonucleotide, particularly as in the second aspect of the present invention, which is at its 3' end complementary to the 3' overhang generated by the restriction endonuclease, and at its 5' end complementary to the 5' end of the first primer. The free end, preferably the 5'-end of the first primer is ligated after phosphorylation by a ligase to the double-stranded structure created upon elongation of the other end, preferably the 3'-end, of the first primer by the polymerase activity and cutting of said structure through a restriction endonuclease. Thus a circular target is obtained which comprises at least part of the target nucleic acid or a sequence which is complementary thereto, preferably the specificity or uniqueness conferring part of the target nucleic acid. It is within the present invention that the first primer may comprise other or additional sequences which are not identical or complementary to the target nucleic acid.

The second primer is preferably complementary to the sequence of the circular target or a part thereof, more preferably to that part of the circular target which is complementary to the part of the target nucleic acid providing for the specificity or uniqueness of the target nucleic acid as defined above, i.e. providing a specific detection of the respective target nucleic acid. It is within the present invention that the sequence of said second primer comprises at least that part of the circular target and thus is identical to the part of the target nucleic acid providing for the uniqueness or for the specific detection of said target nucleic acid. It is within the present invention, that said second primer may comprise additional nucleotides either within, or at each and/or any end of the primer or at both ends. In case the amplification step is part of a detection method whereby polymorphisms are detected and the genotype is determined by the part of the target nucleic acid providing for the specificity or uniqueness of the target nucleotide, the second primer will preferably comprise a sequence which contains the site of the differences of the various genotypes or the site of the mutation or nucleotide polymorphism, preferably single nucleotide polymorphism, to be detected. It is to be understood that due to the design of the primers as disclosed herein, in those embodiments where a circular ligation product is formed, which is formed by or incorporates the target nucleic acid or a copy thereof, the second primer is essentially complementary, preferably complementary to the uniqueness conferring part of the circular ligation product.

The second primer hybridises to the corresponding part of the circular target or circular ligation product, respectively, and, upon addition of buffer, dNTPs and polymerase, a polymerase reaction is performed providing an amplification product, whereby such amplification product consists of tandemly linked copies of the covalently closed circular target comprising the target nucleic acid.

To create an exponential increase of the thus generated amplification product, a third primer is provided in preferred embodiments. The sequence of this third primer, which is also referred to herein as reverse primer, is identical to any part of the circular target or circular ligation product. This embodiment is advantageous insofar as the first primer can be designed such as to have a cassette which is target nucleic acid specific, whereas other part(s) of the circular target or circular ligation product is/are different therefrom and can thus be used in connection with the amplification process of different target nucleic acids. In such case, the third primer can also be used for these various amplification reactions. As the specificity of the reaction is provided by the ligation and the first amplification step mediated by the design of the first and/or second primer and/or any mediating primer as outlined herein, a further discrimination at the level of the third primer is not necessary. However, it is within the present invention that the sequence of the third primer may at least partially be identical to that part of the circular target which provides for the specificity or uniqueness of the target nucleic acid. By doing so, any potentially existing undesired amplification products can be eliminated, at least can be diluted by the further amplification step(s) and therefore, any potentially existing background signal in any detection step may be reduced to an irrelevant level.

It is to be acknowledged that what is disclosed herein in connection with any aspect of the present invention is in principle also applicable to each and any other aspect of the present invention if not indicated to the contrary.

It is within the present invention, that the first, second and third polymerases are the same polymerases, although also different polymerases can be used at the various steps of the method according to the present invention.

The method for the amplification of a target nucleic acid according to the third aspect of the present invention also encompasses an extension oligonucleotide which is preferably phosphorylated at the 5' end and contains a sequence serving for binding of a not target specific third primer or as structural material extending the length of the target molecule, if the target circle is too small for efficient rolling circle amplification. Preferably, said extension oligonucleotide does not comprise a part of the target nucleic acid, particularly not a part which confers the specificity or uniqueness to the target nucleic acid or a nucleic acid which is complementary thereto. In order to ligate the linear extension oligonucleotide with the target nucleic acid, the target nucleic acid is generated, preferably by a digestion process using restriction enzymes and the corresponding double-strand is then melted. The single-stranded target nucleic acid or a nucleic acid which is complementary thereto is ligated to the extension oligonucleotide using a first mediating primer and a second mediating primer to align the participating reactants. The first mediating primer is at least partially complementary to a first stretch of the target nucleic acid or a nucleic acid which is complementary thereto and at least partially complementary to a first stretch of the extension oligonucleotide. The first mediating primer thus comprises two moieties which are individually complementary to said two nucleic acids, namely the target nucleic acid or a nucleic acid which is complementary thereto, and the extension oligonucleotide. The same applies to the second mediating primer. The particular design of the first mediating primer and second mediating primer is within the one skilled in the art and requires a certain number of oligonucleotides to provide for the required stability of the annealed structure. Preferably, the extension oligonucleotide is linked to the target nucleic acid or a nucleic acid which is complementary thereto via the annealing of the first mediating and the annealing of the second mediating primer. Preferably, the thus created double strand is formed by about 10 nucleotides on each side. Without wishing to be bound by any theory, the first mediating primer and the second mediating primer thus act as a clamp nucleic acid to mediate the ligation process of the extension oligonucleotide and the target nucleic acid or a nucleic acid which is complementary thereto. Preferably, these mediating primers are blocked at the 3' end to avoid elongation by the DNA polymerases during the amplification step.

The ligation process is carried out according to standard protocols known to the one skilled in the art and, for example, described in Current protocols in Molecular Biology, editor F.M.Ausubel, Wiley, USA.

The circular target which is present after the ligation step containing at least a sequence which is complementary to at least a part of the target nucleic acid, and the circular ligation product which contains at least part of the target nucleic acid, respectively, is then used as described in connection with the methods according to the first and second aspect of the present invention. Particularly the design of any further primers or extension oligonucleotides used to amplify the target nucleic acid or a nucleic acid which is complementary thereto is applicable in connection with the method according to this third aspect of the present invention as well.

The further steps also comprise the rolling circle replication process initiated by a second primer and the optional use of a third primer so as to further amplify the ligation product and amplification product.

The method according to the fourth aspect of the present invention also realizes the basic principle of having a rolling circle amplification, whereby the circular ligation product contains at least a part of the target nucleic acid or a nucleic acid which is complementary thereto, preferably the part of the target nucleic acid mediating the specificity or uniqueness of the target nucleic acid. In this aspect of the present invention, however, the circular ligation product consists completely of the target nucleic acid or a nucleic acid which is complementary thereto and does not comprise any non-target nucleic acid. According to the third fourth aspect of the present invention, the circularization is facilitated by a mediating primer that is at least partially complementary to a first as well as a last stretch of the target nucleic acid or a nucleic acid which is complementary thereto and thus acts as a clamp nucleic acid to mediate the ligation process. Due to this aspect, the target nucleic acid or a nucleic acid which is complementary thereto should comprise from about 80 to 300 nucleotides. Preferably, the uniqueness conferring part of the target nucleic acid which allows discriminating the target nucleic acid from other nucleic acids preferably has a length of about 20 nucleotides.

According to this aspect of the present invention the target nucleic acid can be generated from a larger nucleic acid, for example by digestion using restriction endonucleases. The thus obtained double-stranded structure is converted into the single-stranded form of the target nucleic acid, whereby, in principle, each of both strands can under these circumstances be regarded as the target nucleic acid as generally applicable to any aspect of the present invention disclosed herein. The mediating primer used in this method anneals to both ends of the target nucleic acid and thus converts the target nucleic acid into a ligase substrate. Again, the ligase reaction can be performed as known to the one skilled in the art. The mediating primer acts in a manner similar to the first and second mediating primer as described in connection with the method according to the third aspect of the present invention. The design principles, particularly about the extent to which one end of the target nucleic acid is complementary to the mediating primer and the extent to which the other end of the target nucleic acid is complementary to the mediating primer is also governed by the same considerations as outlined in connection with the mediating primers described for the second and third aspect of the present invention.

In connection with any method according to any aspect of the present invention it is to be noted that preferably the target nucleic acid is a deoxyribonucleic acid, more preferably a double-stranded deoxyribonucleic acid and most preferably single-stranded deoxyribonucleic acid. However, the target nucleic acid as used in the rolling circle amplification is a single-stranded nucleic acid, more preferably a single-stranded deoxyribonucleic acid. It is known to the one skilled in the art on how to transfer a double-stranded nucleic acid into a single-stranded nucleic acid. Typically, this is performed by melting the respective double-stranded structure by applying elevated temperatures and cooling the respective solution subsequently. Typically, the thus obtained single-stranded ribonucleic acid, preferably deoxyribonucleic acid is the target nucleic acid as referred to herein.

However, the methods described according to any aspect of the present invention are not limited to the amplifications of deoxyribonucleic acid. Rather also ribonucleic acid can be amplified. If said ribonucleic acid is present as a double-stranded form, also double-stranded ribonucleic acid can be separated into individual single-stranded ribonucleic acids by methods known in the art, including but not limited to, melting of the double-stranded structure. A particularly preferred ribonucleic acid which is desirable to be amplified is, among others, mRNA, hnRNA, viral RNA and the like. If the target nucleic acid as used in step a) of the methods according to the various aspects of the present invention is a ribonucleic acid, the ribonucleic acid is subject to a reverse transcription reaction. Such reverse transcription reaction is known to the ones skilled in the art. Preferably, a gene specific primer is used for this purpose. The remaining RNA moiety of the reverse transcription product is removed, for example by RNAse H digestion. In one embodiment starting from the thus obtained single-stranded deoxyribonucleic acid, a second strand is provided. For that purpose a still further primer is used which anneals at least partially to the single-stranded deoxyribonucleic acid. By providing such still further primer, the length of the deoxyribonucleic acid to be generated is defined given the fact that the other end of the strand is defined by the primer used for the synthesis of the first deoxyribonucleic acid strand. The thus obtained single-stranded deoxyribonucleic acid strand can be used as a target nucleic acid in the sense of step a) of the various methods according to any aspect of the present invention. It is particularly preferred to use this kind of single-stranded deoxyribonucleic acid in the method according to the fourth aspect of the method according to the present invention. Some polymerases exhibit terminal transferase activities resulting in additional not target encoded nucleotides at the 3' ends. These will be considered in the design of the mediating primers if necessary.

In an alternative embodiment, the reverse transcription product contains a recognition site for a restriction endonuclease and allows thus to terminate or limit the length of the reverse transcription product. The thus obtained DNA, preferably after digestion with a restriction enzyme, is then used as a target nucleic acid, preferably a single-stranded target nucleic acid, in any method according to any aspect of the present invention. Preferably, the method is the method according to the fourth aspect of the present invention.

In a further embodiment of any aspect of the present invention, more preferably of the fourth aspect of the present invention, the circular target and circular ligation product, respectively, can be generated using the following approach. Based on the target nucleic acid a recombination primer is provided. Such recombination primer comprises two stretches of nucleotides which are essentially complementary to two sites within the target nucleic acid. Upon annealing of the recombination primer to the target nucleic acid and in the presence of respective recombinase activity, a homologous recombination occurs resulting in circularisation of the target nucleic acid thus providing the circular ligation product and the circular target, respectively, as used herein. Starting from this circular structure, the further steps of any of the methods as described in connection with any aspect of the present invention, can be practised for the purpose of amplifying said circular structure.

The amplification product, either the first amplification product obtained or the second amplification product as obtained by the various methods according to the present invention can be detected in further detection steps. The detection of nucleic acids, preferably of oligonucleotides, is known to the one skilled in the art. Preferably, some label is introduced into the amplification product so as to allow for the detection of the nucleic acid or any of its derivatives. This can be done in the various embodiments of the methods according to the present invention by introducing labels into the various primers, preferably into the second and/or third primer. The respective label can be, for example, a radio label or a fluorescence label. A thus labelled primer can be used advantageously for real-time detection of the amplification product(s). Using this kind of labels and labelled compounds, respectively, the amount of fluorescence or radioactivity observed is proportional to the amount of label introduced and thus to the amount of amplification product generated. Due to this relationship both the presence of a respective amplified product as such and the kinetic of the reaction as well as the quantitative determination of the amplification product(s) can be realized. Suitable radio-labels are selected from the group comprising ³²P, ³³P, ³⁵S, ³H, ¹⁴C. Appropriate fluorescent labels are preferentially selected from the group comprising FAM, JOE, HEX, ROX.

It is within the skills of the one of the art to determine whether only one species or two or several of the primer species used in the methods according to the present invention are provided with a respective label. If two or more primers are labelled, it is within the present invention to use different kinds of label for different primers. This allows monitoring the amplification process at a level of the process where the individual primers are used.

A further means for quantitation and real-time monitoring the amplification product is the use of intercalating dyes such as, among others, ethidium bromide, propidium iodide and Cybergreen. Alternatively, pyrophosphate, a reaction product that accumulates during DNA synthesis might be quantitated, e.g. by the addition of a dye (e.g. 1*2Zn, Lee DH, Im JH, Son SU, Chung YK, Hong JI. An azophenol-based chromogenic pyrophosphate sensor in water. J Am Chem Soc. 2003 Jul 2; 125(26): 7752-3) that changes colour in the presence of pyrophosphate.

The amplification process can, in principle, be stopped at any stage and the amount of amplification product(s) then determined. Apart from determining the kind and amount of label incorporated, it is also necessary to discriminate between any incorporated labelled primers and any non-incorporated labelled primers. For such purpose, gel analysis can be performed, whereby the length of the amplification products compared to the length of the labelled primers used in the amplification process is used for this kind of discrimination.

A further method to label the primer(s) used and the amplification product(s) is the use of so-called modified molecular beacons as, for example, described by Nielson M. et al., Nucleic Acids Research, 2002, vol. 30, no. 14, e66. The use of this kind of molecular beacon allows real-time measurement of the amplification product both in a qualitative as well as a quantitative manner. The molecular beacon is a hairpin-shaped hybridization probe such as primer which is equipped with a fluorophore and a fluorescence quencher at either end so that the fluorophore is brought close to the quencher when the free primers adopt a stem-loop structure. Upon hybridization to a target nucleic acid and optionally subsequent incorporation into the amplification product, the fluorophore is separated from the quencher and can emit fluorescence. In other words, upon incorporation of the primer being designed as a molecular beacon as described herein, a fluorescence signal can be detected. Based on the measured values both the kinetics of the amplification process as well as the quantification can thus be monitored and/or detected. Alternatively, self-quenched primers labelled with a single fluorophore (Nazarenko I, Lowe B, Darfler M, Ikonomi P, Schuster D, Rashtchian A. Multiplex quantitative PCR using self-quenched primers labeled with a single fluorophore. Nucleic Acids Res. 2002 May 1; 30(9): e37) might be used.

In a preferred embodiment the methods according to the present invention are run as an isothermal process. However, it is also within the present invention that some steps of the process are not run in an isothermal manner, particularly the amplification steps using the second and particularly the third primer upon generation of an amplification product comprising a variety of amplified target nucleic acid linked to each other or linked to each other but separated through a nucleic acid sequence stemming from the circular target and the circular ligation product, respectively, in the rolling circle amplification process.

In preferred embodiments of the present invention, the polymerase is typically a DNA polymerase and the primers are preferred DNA primers, particularly if not indicated to the contrary or obvious to the one skilled in the art. DNA polymerases which can advantageously be used in connection with the rolling circle amplification part of the present invention are DNA polymerase III, pol III-type DNA polymerase and pol I-type DNA polymerase that are capable of strand displacement polymerisation like e.g. Bst large fragment (Thomas DC, Nardone GA, Randall SK. Amplification of padlock probes for DNA diagnostics by cascade rolling circle amplification or the polymerase chain reaction. Arch Pathol Lab Med. 1999 Dec;123(12):1170-6), Vent exo⁻ (Viguera E, Canceill D, Ehrlich SD. In vitro replication slippage by DNA polymerases from thermophilic organisms. J Mol Biol. 2001 Sep 14;312(2):323-33), Deep vent exo- (Cline J, Braman JC, Hogrefe HH. PCR fidelity of pfu DNA polymerase and other thermostable DNA polymerases. Nucleic Acids Res. 1996 Sep 15;24(18):3546-51), KlenowFragment of DNA polymerase I (Walker GT, Fraiser MS, Schram JL, Little MC, Nadeau JG, Malinowski DP. Strand displacement amplification--an isothermal, in vitro DNA amplification technique. Nucleic Acids Res. 1992 Apr 11;20(7):1691-6) or phi29 (Nelson JR, Cai YC, Giesler TL, Farchaus JW, Sundaram ST, Ortiz-Rivera M, Hosta LP, Hewitt PL, Mamone JA, Palaniappan C, Fuller CW. TempliPhi, phi29 DNA polymerase based rolling circle amplification of templates for DNA sequencing. Biotechniques. 2002 Jun;Suppl:44-7).

It is within the skills of the one of the art how to use this kind of polymerases and to perform respective polymerase reactions.

It is within the present invention that the individual polymerase used in each step of any method according to the present invention is different from the polymerase used in a different step of such method. It is, however, also within the present invention that the various polymerases used in a method according to the present invention are either the same or of the same type.

It is to be noted that actually any nucleic acid, preferably any deoxyribonucleic acid can act as a target nucleic acid. Preferably, the target nucleic acid, however, comprises a minimum length of about 40 to 50 nucleotides.

As used herein, the term "complementary" shall mean in preferred embodiments that a first and a second nucleic acid can hybridize and/or anneal to each other. Preferably such hybridization is mediated through Watson-Crick base pairing or other mechanisms known to the ones skilled in the art. Most preferably, the hybridization and the underlying base pairing are perfect. However, it is also within the present invention that the base pairing is not perfect but exhibits one or more mismatches. The extent to which this kind of mismatches still allows the carrying out of the respective method is obvious and known to the one skilled in the art. It is therefore within a further preferred embodiment that a first nucleic acid is complementary to a second nucleic acid such as any primer or any extension oligonucleotide, or vice versa, whereby the first and the second nucleic acid, can hybridize to each other, at least under stringent conditions such as described in Methods in molecular Biology, Vol 15, PCR Protocols, ed: Bruce A. White, Humana Press, Totowa, New Jersey. As used herein, the term "at least partially complementary" means in a preferred embodiment of the various aspects of the present invention that a part of a first nucleic acid is complementary to at least a part of another nucleic acid. Such part can be a single nucleotide or a plurality of nucleotides. The term "at least partially complementary" also comprises the embodiment where each and all of the respective nucleotides are complementary to each other.

As used herein the term "identical" shall mean in preferred embodiments of the various aspects of the present invention that a first and a second nucleic acid would, if one of said nucleic acids was present in its complementary form, would be complementary as defined herein. In a further embodiment the term identical is to be understood that the two nucleic acids which are identical share a homology at the nucleotide level of 80, 85, 90, 95, 96, 97, 98, 99 and/or 100%.

Although the methods for amplifying a target nucleic acid as well as any other methods described herein are preferably *in vitro* methods, the various enzymatic activities and primers described in connection with said methods according to the present invention can also be used for the design or manufacture of a kit, whereby such kit is used for performing the respective methods *in vivo* and/or *in vitro.*

In a further aspect the present invention is related to a method for the detection of a polymorphism, preferable a single nucleotide polymorphism. Methods for the detection of polymorphism are known to the one skilled in the art and, for example, described in Koeken A, Cobbaert C, Quint W, van Doorn LJ. Genotyping of hemochromatosis-associated mutations in the HFE gene by PCR-RFLP and a novel reverse hybridization method. Clin Chem Lab Med. 2002 Feb;40(2):122-5; Aslanidis C, Nauck M, Schmitz G. High-speed prothrombin G-->A 20210 and methylenetetrahydrofolate reductase C-->T 677 mutation detection using real-time fluorescence PCR and melting curves. Biotechniques. 1999 Aug;27(2):234-6, 238; and Meyer M, Kutscher G, Vogel G. Simultaneous genotyping for factor V Leiden and prothrombin G20210A variant by a multiplex PCR-SSCP assay on whole blood. Thromb Haemost. 1999 Jan;81(1):162-3.

Polymorphisms are known to be of highly relevance in the medical field. It is known that the efficacy of quite a number of pharmaceutically active compounds as well as side effects of drugs strongly depend on the genetic background of the individual patient. This resides, among others, in the receptor pattern or enzyme pattern of the individual patient. Additionally, single nucleotide polymorphisms are known to be relevant insofar as a number of hereditary diseases are known where a single point mutation is responsible for the disease observed. Also there are polymorphisms which may occur in diseased tissue, for example in cancer patients. Under all and any of these conditions, it is essential to determine whether a sample comprises such polymorphism and single nucleotide polymorphism. Typically, the respective stretch of the relevant gene where a polymorphism is observed in a population is rather short. Therefore, it is sufficient to amplify only a comparatively short stretch of the respective gene to be investigated for the particular purpose. Preferably the length of such stretch of a gene is 60 - 300 bp. This stretch is also referred to herein as target nucleic acid or part thereof which provides for the uniqueness of the gene.

To allow for the selective amplification, it is essential to amplify only those target nucleic acids actually exhibiting the single nucleotide polymorphism as otherwise a false positive result would be generated which could have detrimental effects. A particular scenario arises in case the target nucleic acid comprises more than one single nucleotide polymorphism or if highly homologous genes exist, like in gene families. Under these circumstances the design of the second and third primer allows the simultaneous selective detection of two single nucleotide polymorphisms, if they are made such that the 3' base of the respective primer corresponds to the position of the respective SNP. Additional single nucleotide polymorphisms may be simultaneously detected by choosing restriction endonucleases that digest the target only if the single nucleotide polymorphism is present. Since the design is made in a way that the digest is essential for the formation of the circular target and the formation of the circular target or the circular ligation product, respectively, is essential for the amplification, no product is generated in the absence of the single nucleotide polymorphism. More preferably, this approach is realized in the method according to the third and fourth aspect of the present invention. As the ends of the resulting DNA fragments are not complementary to the first and second mediating primers in case of the method according to the third aspect and to the single mediating primer of the method according to the fourth aspect, the DNA fragments are not circularized and may therefore not be subject to rolling circle amplification.

In a further aspect the present invention is related to a method for diagnosis, whereby the method comprises an amplification step. This amplification step may be carried out according to any aspect of the method for the amplification of a target nucleic acid according to the present invention. As outlined already earlier in connection with the method for the detection of polymorphisms according to the present invention, the diagnostic approach particularly related to or based on single nucleotide polymorphisms, but also related to other scenarios where a condition to be diagnosed is manifested within a certain target nucleic acid, can be addressed using the technical teaching of the present invention (Silao CL, Padilla CD, Matsuo M. A novel deletion creating a new terminal exon of the dihydrolipoyl transacylase gene is a founder mutation of Filipino maple syrup urine disease. Mol Genet Metab. 2004 Feb; 81 (2): 100-4). The same applies also to genotyping: Werner FA, Durstewitz G, Habermann FA, Thaller G, Kramer W, Kollers S, Buitkamp J, Georges M, Brem G, Mosner J, Fries R. Detection and characterization of SNPs useful for identity control and parentage testing in major European dairy breeds. Anim Genet. 2004 Feb; 35(1): 44-49. , genomic mapping: Lustig LR, Peng H. Chromosome location and characterization of the human nicotinic acetylcholine receptor subunit alpha (alpha) 9 (CHRNA9) gene. Cytogenet Genome Res. 2002; 98(2-3): 154-9. , DNA sequencing Ehlers A, Beck S, Forbes SA, Trowsdale J, Volz A, Younger R, Ziegler A. MHC-linked olfactory receptor loci exhibit polymorphism and contribute to extended HLA/OR-haplotypes. Genome Res. 2000 Dec;10(12):1968-78. und synthesis of DNA probes: Wende H, Volz A, Ziegler A Extensive gene duplications and a large inversion characterize the human leukocyte receptor cluster. Immunogenetics. 2000 Jul;51(8-9):703-13.] As obvious for the one skilled in the art from the description of the various methods according to the different aspects of the present invention, the specificity of the amplification process is crucial to any of these methods and allows to increase the sensitivity and thus reliability of the respective methods.

Any of the methods according to the present invention may be subject to a high throughput application. This resides in the fact that any of said methods according to the present invention can, at least to the extent the amplification process is concerned, be run as a real-time process including a real-time detection system which allows that the reaction vessel may be discarded at the end of the reaction thus avoiding cross contamination. If the reaction vessels are used several times or the reaction product shall be subject to a control via gel analysis, the restriction enzymes used in some embodiments of the methods according to the present invention can be selected such that the recognition site of at least one of these enzymes is destroyed through the ligation process. As these products can no longer be cut by the very same restriction endonuclease, no new cycles or rings can be formed so that subsequent rolling circle amplification reactions would not result in false positive results despite possible cross-contaminants or carryover contamination.

The invention is further illustrated by reference to the figures and examples from which further features, embodiments and advantages of the present invention may be taken. More particularly,
- Fig. 1A to 1H: shows the principle reaction steps of the method according to the first aspect of the present invention;
- Fig. 2: shows some of the reaction steps of the method according to the second aspect of the present invention, whereby these steps are those discriminating the method according to the second aspect from the method according to the first aspect of the present invention;
- Fig. 3: shows essential steps of the method for the amplification of a target nucleic acid according to the third aspect of the present invention;
- Fig. 4: shows some essential steps of the method for the amplification of a target nucleic acid according to the fourth aspect of the present invention;
- Fig. 5: shows some essential steps of a method according to the fourth aspect of the present invention, whereby the target nucleic acid is formed from an mRNA;
- Fig. 6: shows the result of an amplification step of a fragment of the factor V gene using different dilutions of the target, expressed as fluorescence as a function of time in minutes;
- Fig. 7: shows the analysis of genomic DNA comprising about 3.000 copies compared to 30 copies of the factor V wild type allele using a wild type specific assay. The control contains again 3.000 copies of the factor V wild type allele but was analysed with the Factor V Leiden specific variant of the assay, expressed as fluorescence as a function of time in minutes;
- Fig. 8 and 9: show the result of an assay for the detection of Factor V from donor samples, expressed as fluorescence as a function of time.

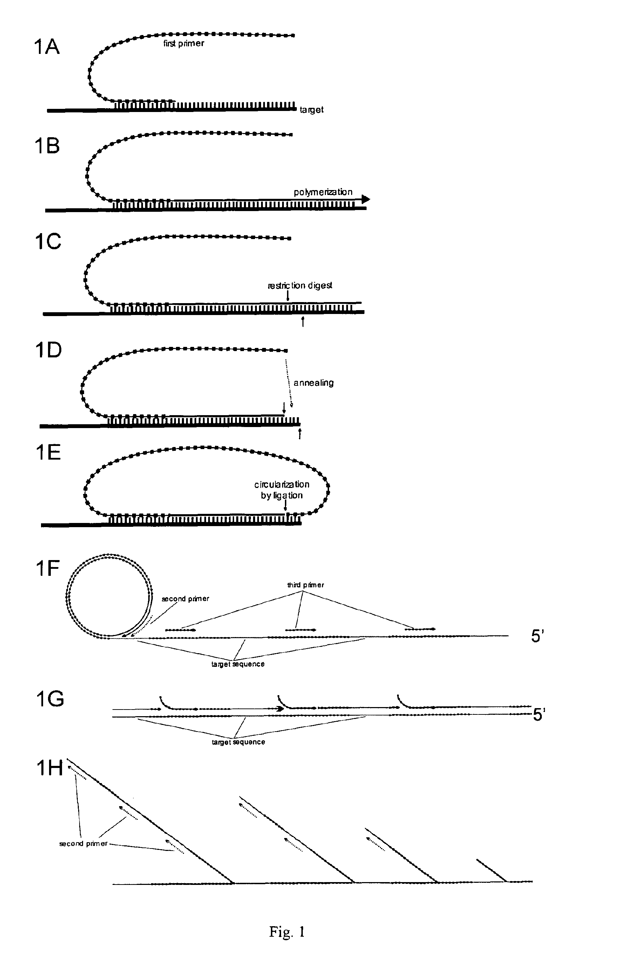

Fig. 1A shows a single-stranded target nucleic acid, which is a DNA, to which the first primer, supplied in a 5' phosphorylated form, is partially hybridized due to sequence complementarity between the 3'-end of the first primer and the target nucleic acid. In Fig. 1B the first primer is extended through the activity of a polymerase reaction upon addition of dNTPs, leading to a primer/target nucleic acid chimera which is double-stranded in the downstream part and comprises a recognition site for a restriction enzyme. In the next step as depicted in Fig. 1C, said double-stranded part of the nucleic acid is cleaved by the restriction enzyme leaving an overhang, more particularly a 5' overhang created by the target nucleic acid which allows the 5'-end of the first primer which is complementary to this overhang, to anneal thereto (Fig 1D). The thus annealed and 5' phosphorylated first primer is ligated to the copy of the target sequence thus forming the circular target (Fig. IE) which is used in the rolling circle amplification process. Upon creation of the circular target a second primer is used which is also referred to as forward primer and is specific insofar as it corresponds to the target nucleic acid or a part thereof. In the present embodiment, the circular target comprises a complementary copy of a part of the target nucleic acid, particularly that part of the target nucleic acid which is conferring the specificity or uniqueness of the target nucleic acid. This specificity or uniqueness may be defined such that the respective specificity or uniqueness conferring target nucleic acid comprises the single nucleotide polymorphism or the particular mutation to be detected in a respective process or method comprising an amplification step for the thus defined target nucleic acid. Additionally, the circular target comprises a sequence of nucleotides which are different from the target nucleic acid so as to allow for the formation of a circular structure rather than the formation of a completely double-stranded structure (Fig 1D). As depicted in Fig. 1F a first amplification product is created due to the rolling circle amplification process, whereby the target nucleic acid copies are separated from each other through complementary copies of that part of the first primer which is different from the target nucleic acid. To this linear amplification product a third primer is added. As depicted in Fig. 1 F this primer is complementary to a part of the amplification product, which is also referred to herein as the first amplification product. Preferably, the third primer is complementary to either the target nucleic acid or identical to the part of the first primer different from that target nucleic acid.

The annealed third primer is extended forming the second amplification product (Fig 1F und G).

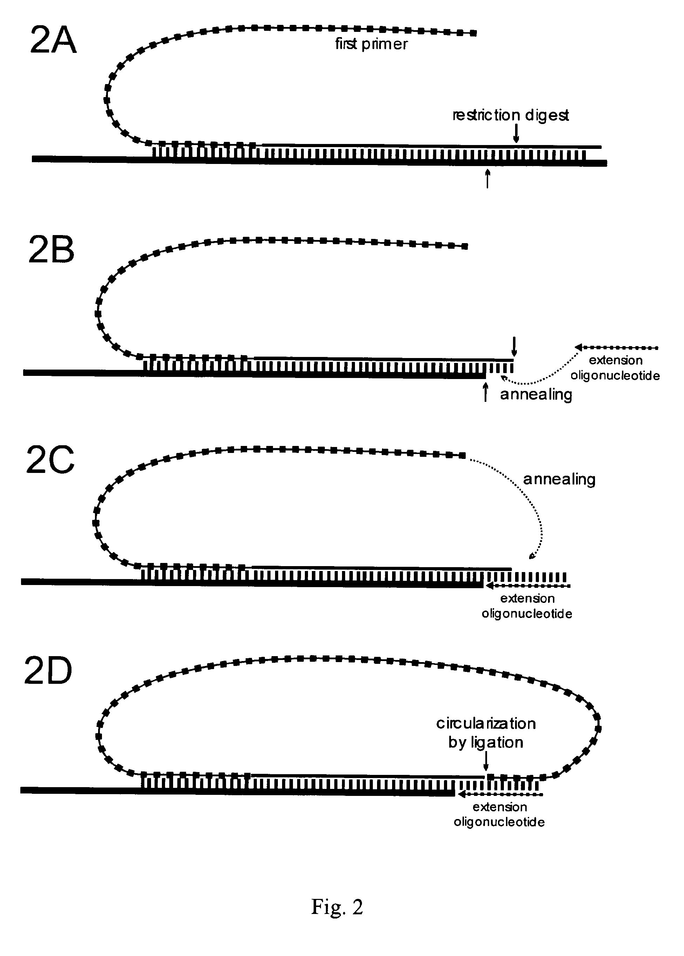

Fig. 2 shows some of the reaction steps of the method according to the second aspect of the present invention, whereby these steps are those discriminating the method according to the second aspect from the method according to the first aspect of the present invention. More particularly, in Fig. 2A the first primer is shown annealed to the target nucleic acid and extended through the activity of the polymerase using the target nucleic acid as matrix. In principle, polymerase activity will last until the end of the target nucleic acid is reached, however, may be terminated earlier. As in the method according to the first aspect of the present invention, the thus elongated double-stranded target nucleic acid comprises a recognition site for a restriction enzyme. In the method according to the second aspect, however, the restriction enzyme generates a 3' overhang (Fig. 2B). To the thus created 3' overhang an extension oligonucleotide is annealed through base pairing thus extending the 5' end of the target nucleic acid generated upon the cleavage by the restriction endonuclease. One end of the extension oligonucleotide is thereby complementary to the 3' overhang generated by the endonuclease and allows for said annealing. Another part of the extension oligonucleotide is complementary to the 5' end of the first primer. Thus, the function of the extension oligonucleotide is similar to the one of the mediating primers as disclosed herein and the respective design principles are applicable to this kind of extension oligonucleotide as well.

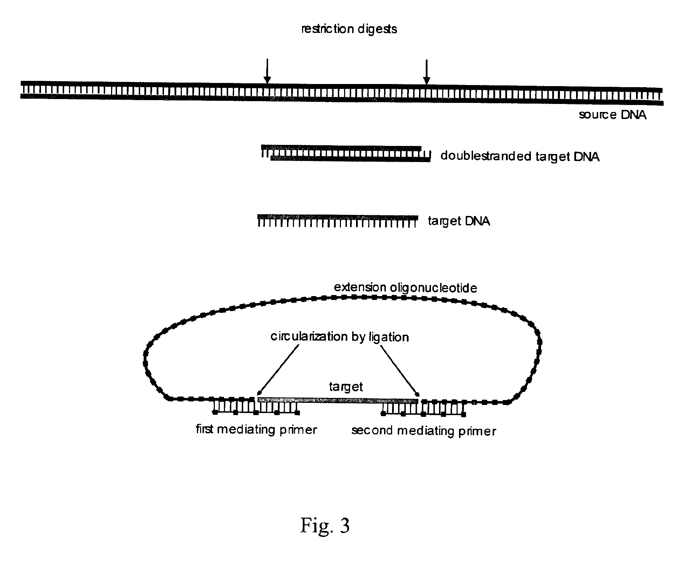

Fig. 3 shows the essential steps of a method for the amplification of a target nucleic acid according to the third aspect of the present invention. More particularly, a double-stranded nucleic acid which comprises the target nucleic acid, i.e. a single-stranded DNA as part thereof, is prepared by digesting said nucleic acid using one or two restriction enzymes. The thus created double-stranded DNA structure is separated into the individual strands and each individual strand can, in principle, form a target nucleic acid as used in the various embodiments of the methods for the amplification of a nucleic acid according to the present invention. The target nucleic acid is ligated to the ends of the linear extension oligonucleotide, which is supplied in a 5' phosphorylated form. This ligation is mediated by two different primers, namely the first mediating primer and the second mediating primer. One part or end of the first mediating primer is essentially complementary to a first end of the target nucleic acid and the other end is essentially complementary to a first end of the extension oligonucleotide. The second mediating primer exhibits basically the same design, whereby one part or end of said second mediating primer is complementary to a second end of the target nucleic acid and the other end of the second mediating primer is essentially complementary to the second end of the extension oligonucleotide. The mediating primers anneal to the first primer and the target molecule in a way that the extension oligonucleotide and the target molecule are directly adjacent to each other, i.e. no base is missing in between, at both ends, respectively. In order to avoid side products in the latter amplification steps, the mediating primers may be blocked (e.g. by phosphorylation or additional non fitting bases) at the 3' end or used also as secondary primers. It is to be acknowledged that the first end of the target nucleic acid is to be ligated to the first end of the extension oligonucleotide and the second end of the target nucleic acid is to be ligated to the second end of the extension oligonucleotide. Due to this design of both the extension oligonucleotide and the first mediating primer and the second mediating primer, respectively, the extension oligonucleotide can be ligated to the target nucleic acid forming a partially double-stranded circular structure, the circular target. This partially double-stranded structure can be further reacted using a polymerase and a first primer, whereby the first primer is at least partially complementary to the target nucleic acid, preferably the first primer is complementary to a part of the target nucleic acid contained in the circular target. It is within the present invention that the first and/or second mediating primer is/are used as the first primer for performing the polymerase reaction creating the first amplification product, if the second primer is designed in a way that ensures specificity of the reaction by annealing to the uniqueness mediating part of the first amplification product.

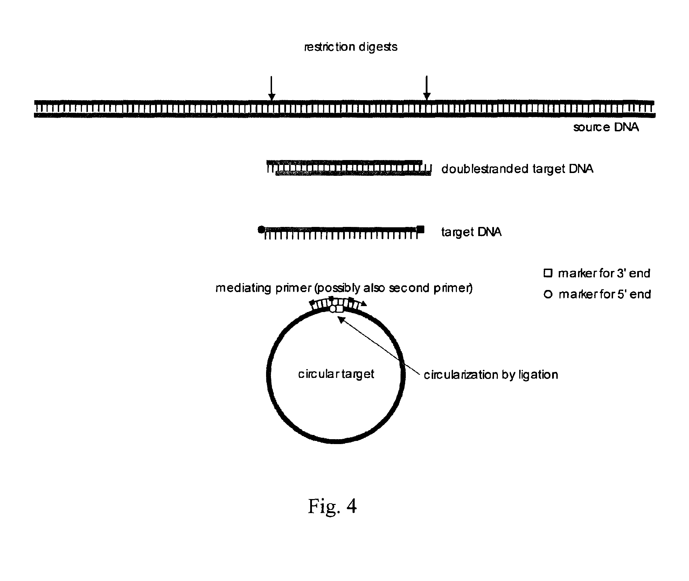

Fig. 4 shows essential steps of the method for the amplification of a target nucleic acid according to the fourth aspect of the present invention. More particularly, the target nucleic acid is prepared from a nucleic acid comprising the target nucleic acid which is typically present as a double-stranded DNA. From said double-stranded DNA the target nucleic acid or a larger fragment which comprises the actual target nucleic acid is prepared. Preferably, such fragment is prepared by digesting said nucleic acid using one or two restriction enzymes. The thus created double-stranded target nucleic acid is converted into the single-stranded form creating the target nucleic acid as used in the various embodiments of the methods according to the present invention. The single-stranded target nucleic acid is in the present embodiment that big that it allows direct circularization which is mediated by a mediating primer. Said mediating primer comprises a first end which is essentially complementary to a first end of the target nucleic acid and a second end which is essentially complementary to the second end of said target nucleic acid. The function of this mediating primer is thus similar to the one of the first mediating and second mediating primer, respectively, as described in connection with the method for the amplification of a target nucleic acid according to the third aspect of the present invention. This primer may subsequently act as a second primer for amplification purposes. It is, however, also within the scope of the present invention that the mediating primer is used in a form blocked at the 3' end to avoid side products during the amplification process and consequently, a further primer is added which acts as a second primer, i.e. initiates the rolling circle amplification.

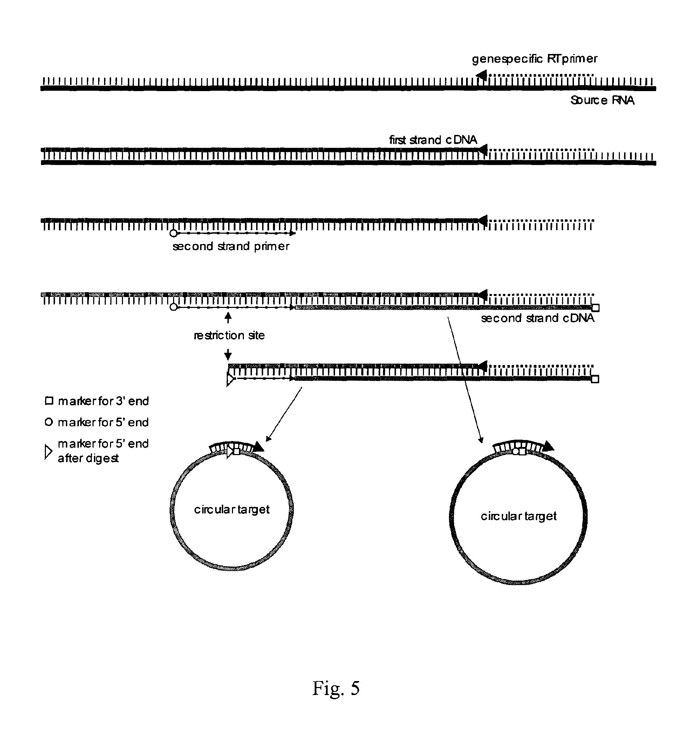

Fig. 5 shows some essential steps of the method according to the fourth aspect of the present invention, whereby the target nucleic acid used in the amplification process is derived from an mRNA. More particularly, Fig. 5A shows the mRNA to which a gene specific primer is annealed which is extended through a reverse transcriptase (Fig. 5B). The thus generated double-stranded structure is melted so as to provide the two corresponding single-stranded nucleic acids, one being a ribonucleic acid and the other one being a deoxyribonucleic acid. Alternatively, the RNA strand might be removed by RNAse H digestion. A further primer is added to the deoxyribonucleic acid strand and, upon annealing to said strand and extension, a double-stranded structure is generated. The synthesis of the double-strand will be stopped at the end of the reverse primer thus creating a defined downstream end. Since both ends of this second strand are clearly defined by the two primers used, the second strand may be used after separation from the first strand in any of the methods according to the present invention.

Alternatively, the double-stranded cDNA structure can be used as source for any of the methods according to the present invention.

### Example 1: Detection of a factor V fragment in a serial dilution assay

### Source:

As source, a fragment of the human factor V wild type gene, terminated by the primers F5-Mut-for_out (5' CCACAGAAAATGATGCCCAG) and F5-Mut-rev2 (5' GCCCCATTATTTAGCCAGGAG) and cloned in pCR4 (Invitrogen) was used. The isolated plasmid (~100ng/µl) was diluted in TE containing 5ng/µl salmon sperm DNA. This blocking DNA was added in order to avoid loss of the plasmid due to unspecific adsorption to the wall of the reaction vessel. Serial dilutions, starting with 1:10000 (~10pg Plasmid DNA/µl) in 1:10 steps were used for target generation.

### Target generation:

A 10 µl digest was performed at 37°C for 30 minutes. The reactions contained 0.3 µl source DNA of the respective dilutions, 10 U Dde1 and 10 U MspI in 1 x Buffer K containing BSA (Promega, Mannheim). Enzymes were inactivated and DNA was denatured for 1 minute at 96°C. The following sequence with a length of 116 bp was set free by this reaction:

CATGAGAGACATCGCCTCTGGGCTAATAGGACTACTTCTAATCTGTAAGAGCAGA
TCCCTGGACAGGCAAGGAATACAGGTATTTGTCCTTGAAGTAACCTTTCAGAAA
TTCTGA

### Circular target generation:

The reaction was cooled to 25°C and 1 µl ligation mix, containing 20 fmol/µl F5-Loop_V3L (5'-GAAATTCTGACATGAGAGACAT), 1nmol/µl ATP and 0,4 U T4-ligase (NEB, Frankfurt/Main) in 1 µl diluent buffer A (NEB, Frankfurt/Main), was added. After thoroughly mixing the reactants, the reaction was incubated for 15 minutes at 30°C to form the circular target still 116 bases in size.

### Rolling circle amplification:

The reaction mixture was put on ice and 30 µl of rolling circle mixture, containing 7,5 nmol dATP, dTTP, dGTP, dCTP each, 5 pmol second primer (wild type specific: F5_norm 5'-AGAGCAGATCCCTGGACAGGCG, F5 Leiden-variant: F5-Leiden 5'-AGAGCAGATCCCTGGACAGGCA), 5 pmol labelled third primer (F5-FRET 5'-6FAM-ATAGCGTTTTTACGCTA-T+Dabcyl-TAAGCCCAGAGGCGATGTCTCTCA) and 4 U Bst-polymerase (NEB, Frankfurt/Main) in Addon-buffer (36 mM Tris Cl pH8,6, 25 mM NH₄SO₄, 4% Triton X100) were added. After mixing thoroughly, the reaction was transferred into a real-time cycler (Rotorgene, Corbett Research, Mortlake) and incubated at 65°C. The fluorescence was measured at 510 nm after excitation at 470 nm every minute.

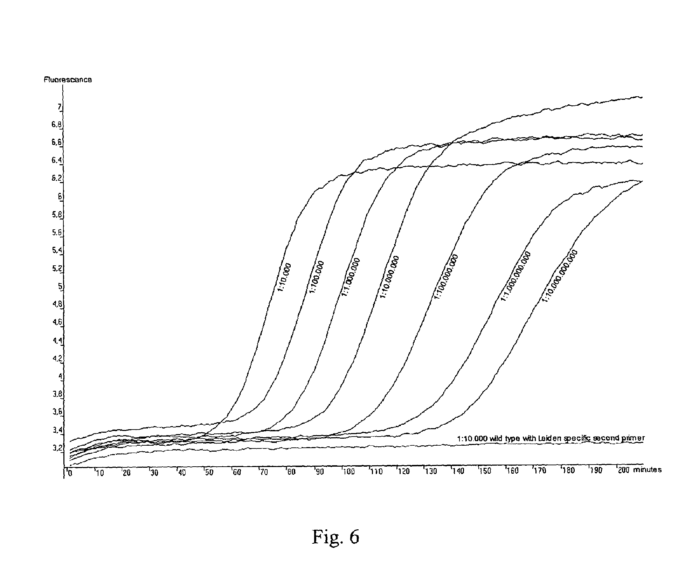

The results of this experiment are shown in Fig. 6.

As maybe taken from Fig. 6 the serial dilution assay starting from about 3 pg of purified plasmid corresponding to about 2.3 million copies of the respective fragment gave the maximum reaction in said assay expressed as fluorescence after the shortest time. Any serial dilusion of 1:10 resulted in an increase of time required to reach a similar level of fluorescence. The maximum dilution depicted in Fig. 6 corresponds to about two copies which were presented in the reaction assay. This confirms the high sensitivity of the amplification methods according to the present invention. The high specificity is confirmed by a reaction using also 2.3 million copies wild type F5 gene, but conducting the reaction with the F5-Leiden specific primer. This reaction does not generate any fluorescence.

### Example 2: Discrimination between genomic wildtype factor V DNA and genomic factor V Leiden mutation DNA

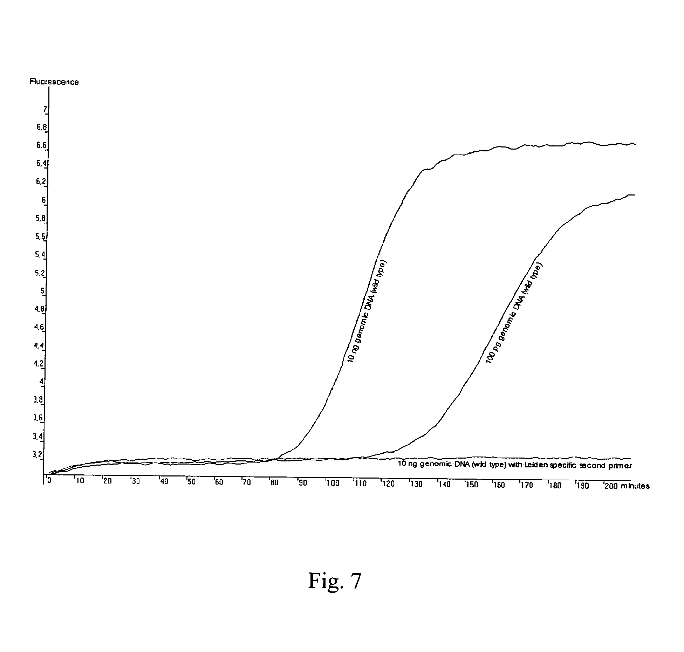

This analysis was done exactly as example 1, but the diluted plasmids were replaced by 10 ng genomic DNA of a healthy donor homozygous for the wild type allele. The result of this experiment is depicted in Fig. 7. As maybe taken from Fig. 7 the above described assay could discriminate between an amplification of the genomic wild-type factor V DNA and the genomic factor V Leiden mutation DNA. The wild-type as well as the factor V Leiden specific DNA amplification were started using 10 ng genomic DNA which corresponds to about 3,000 copies or a dilution of 1:100 which corresponds to about 30 copies of the genomic wild-type factor V DNA. The respective lines in Fig. 7 represent the kinetics expressed as time over fluorescence. Although the difference between the Leiden mutation and the wild-type sequence corresponds to a single nucleotide only, the wild type DNA fragment was not amplified when using the Leiden specific second primer which confirms the high specificity of the amplification process.

### Example 3: Detection of wild type factor V DNA and factor V Leiden mutation DNA in patient samples

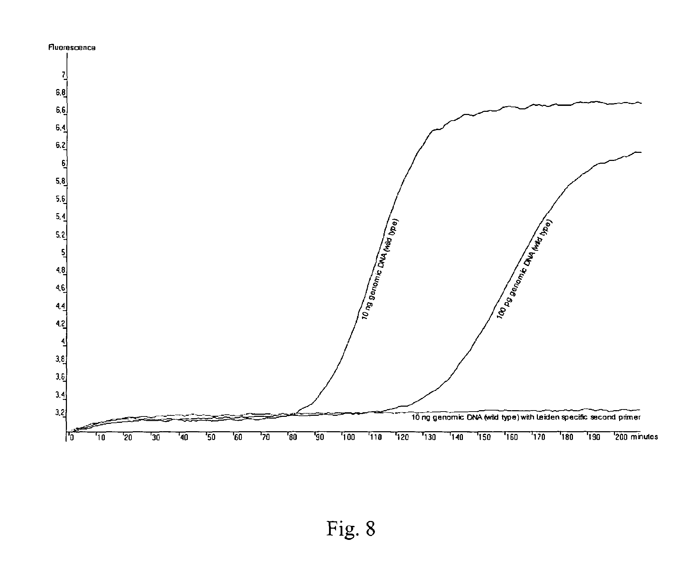

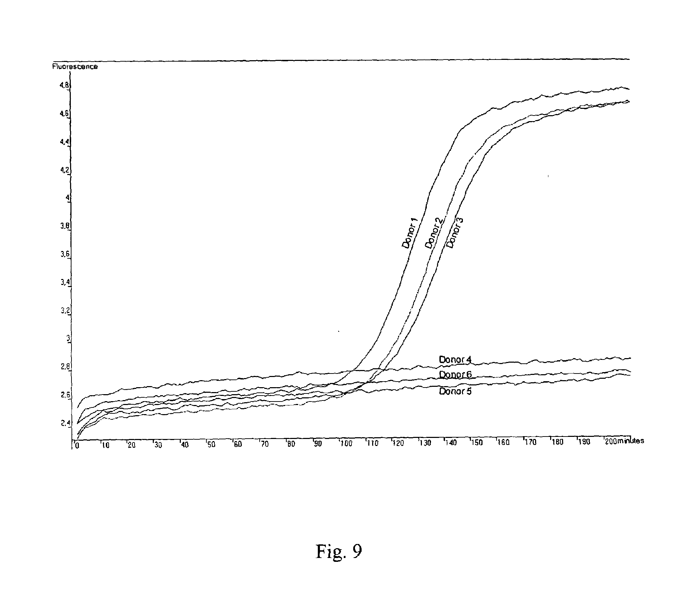

This analysis was done exactly as example 1 and 2, but the samples were replaced by 20 ng genomic DNA of 6 donors which were pretyped by a different (PCR/restriction digest) method. Donor 1 was homozygous and Donors 2 and 3 were heterozygous for the factor V Leiden allele. All donors were tested for the presence of the wild type allele (Fig. 8) and for the presence of the Leiden allele (Fig. 9) using the embodiments as described herein. As may be taken from Fig. 8 only the line of donor 1 did not result in the generation of the reaction product, and therefore a fluorescence signal as a function of time using the wild-type specific primer.

Using the primer, i.e. the second primer in the various embodiments as described herein, having specificity for the factor V Leiden mutation, only donors 1, 2 and 3 (Fig. 9) which were the only donors containing the respective allele were positive. Due to the fact that donor no. 1 shows reactivity only in Fig. 9 but not in Fig. 8, it might be concluded that he is homozygous for the factor V Leiden allele, whereas donors 2 and 3 show reactivity in both reactions and are therefore heterozygous for the Leiden mutation.

The features of the present invention disclosed in the specification, the claims and/or the drawings may both separately and in any combination thereof be material for realizing the invention in various forms thereof.

## Claims

1. Method for the amplification of a target nucleic acid, whereby the method comprises the following steps:
a) providing the target nucleic acid;
b) providing a first primer, whereby the primer is at least partially complementary to a part of the target nucleic acid;
c) providing a polymerase;
d) reacting the target nucleic acid, the primer and the polymerase providing a reaction product, whereby the reaction product comprises a restriction site for a restriction endonuclease;
e) digesting the reaction product of step d) with the restriction endonuclease providing a digestion product;
f) ligating the 5' end of the first primer with the digestion product producing a circular target;
g) providing a second primer, whereby the second primer is at least partially identical to the target nucleic acid;
h) reacting the circular target with the second primer and a second polymerase providing a first amplification product;
i) optionally providing a third primer, whereby the sequence of the third primer is at least partially identical to a part of the first primer or complementary to the target nucleic acid; and
j) optionally reacting the second and the third primer with the first amplification product or the circular target and a third polymerase to provide a series of second amplification products.

2. The method according to claim 1, whereby the 5'-end of the first primer is at least partially complementary to the 5' overhang of the digestion product of step e)

3. The method according to any of claims 1 and 2, whereby the 5' end of the first primer is ligated to the 3' end of the reaction product generated in steps d) and e).

4. A method for the amplification of a target nucleic acid, whereby the method comprises the following steps:
a) providing the target nucleic acid;
b) providing a first primer, whereby the primer is at least partially complementary to a part of the target nucleic acid;
c) providing a polymerase;
d) reacting the target nucleic acid, the primer and the polymerase providing a reaction product, whereby the reaction product comprises a restriction site for a restriction endonuclease;
e) digesting the reaction product of step d) with the restriction endonuclease providing a digestion product;
f) providing an extension oligonucleotide, whereby one end of the extension oligonucleotide is at least partially complementary to the 3' overhang of the digestion product of step e) annealing such extension oligonucleotide to the digestion product of step e), providing an annealed digestion product;
g) ligating the 5' end and 3' end of the annealed digestion product producing a circular target;
h) providing a second primer, whereby the second primer is at least partially identical to the target nucleic acid;
i) reacting the circular target with the second primer and a second polymerase providing a first amplification product;
j) optionally providing a third primer, whereby the sequence of the third primer is at least partially identical to a part of the first primer or complementary to the target nucleic acid; and
k) optionally reacting the second and the third primer with the first amplification product or the circular target and a third polymerase to provide a series of second amplification products.

5. The method according to claim 4, whereby the 5' end of the first primer is at least partially complementary to a stretch, preferably an end, more preferably the 3' end of the extension oligonucleotide.

6. Method for the amplification of a target nucleic acid comprising the following steps:
a) providing the target nucleic acid;
b) providing an extension oligonucleotide;
c) providing a first mediating primer, whereby the first mediating primer is at least partially complementary to a first stretch of the target nucleic acid and at least partially complementary to a first stretch of the extension oligonucleotide;
d) providing a second mediating primer, whereby the second mediating primer is at least partially complementary to a second stretch of the target nucleic acid and at least partially complementary to a second stretch of the extension oligonucleotide;
e) reacting the extension oligonucleotide, the first mediating primer and the second mediating primer and the target nucleic acid with a ligase creating a circular ligation product of the target nucleic acid and the first primer;
f) providing a first primer, whereby the first primer is at least partially complementary to the target nucleic acid;
g) reacting the circular ligation product with the first primer and a first polymerase providing a first amplification product;
h) optionally providing a second primer, whereby the sequence of the second primer is at least partially identical to a part of the extension oligonucleotide or of the target nucleic acid; and
i) reacting the first and second primer with the first amplification product or the circular ligation product and a second polymerase to provide a series of second amplification products.

7. The method according to claim 6, whereby the target nucleic acid is created by cutting of a nucleic acid molecule with one or several restriction enzymes and subsequent denaturation, whereby the target nucleic acid preferably comprises about 40 to 150 nucleotides.

8. Method for the amplification of a target nucleic acid comprising the following steps:
a) providing a target nucleic acid;
b) providing a mediating primer, whereby the first end of the mediating primer is at least partially complementary to the first end of the target nucleic acid and the second end of the mediating primer is at least partially complementary to the second end of the target nucleic acid;
c) reacting the target nucleic acid and the mediating primer with a ligase providing a circular ligation product;
d) reacting the circular ligation product with a DNA polymerase, whereby the mediating primer is annealed to the circular ligation product providing an amplification product;
e) optionally providing a second primer, whereby the sequence of the second primer is at least partially identical to the target nucleic acid; and
f) reacting the mediating and second primer with the amplification product or the ligation product and a first polymerase to provide a series of second amplification products.

9. The method according to any of claims 1 to 8, whereby any of the primers, mediating primers and/or extension oligonucleotides used comprises a label.

10. The method according to any of claims 1 to 9, whereby any of the first, second and third polymerase is a DNA polymerase.

11. The method according to any of claims 1 to 10, whereby the target nucleic acid is derived from a ribonucleic acid by the following steps:
a) providing a ribonucleic acid, preferably an mRNA;
b) providing a primer which is at least partially complementary to the ribonucleic acid of step a);
c) reacting the ribonucleic acid and the primer with a reverse transcriptase providing a reverse transcription product; and
d) removing the RNA moiety of the reverse transcription product, preferably by RNAse H digestion, providing a single stranded deoxyribonucleic acid.

12. The method according to claim 11, whereby the method further comprises the following steps:
e) providing a further primer which anneals at least partially to the single stranded deoxyribonucleic acid;
f) performing a DNA synthesis creating a double stranded nucleic acid; and
g) using one strand of the double stranded nucleic acid as the target nucleic acid.

13. Use of the amplification process comprising the method according to any of claims 1 to 12 in a method for diagnosis, phase determination, genotyping, genomic mapping, DNA sequencing, synthesis of DNA probes and/or cloning.
